Europäisches Patentamt

European Patent Office

Office européen des brevets

(11) Numéro de publication: **0 125 159**
**B1**

(12) **FASCICULE DE BREVET EUROPEEN**

(45) Date de publication du fascicule du brevet: **09.03.88**

(21) Numéro de dépôt: **84400719.5**

(22) Date de dépôt: **11.04.84**

(51) Int. Cl.⁴: **C 12 N 15/00**, A 01 H 1/02

(54) **Procédé d'obtention d'hybrides somatiques de luzernes par fusion de protoplastes.**

(30) Priorité: **13.04.83 FR 8306024**

(43) Date de publication de la demande:
**14.11.84 Bulletin 84/46**

(45) Mention de la délivrance du brevet:
**09.03.88 Bulletin 88/10**

(84) Etats contractants désignés:
**AT BE CH DE FR GB IT LI LU NL SE**

(56) Documents cités:
**GB-A-1 310 119**
**US-A-4 300 310**

**BIOSCIENCE, vol. 30, no. 1, janvier 1980 (US), G.F. SPRAGUE et al.:"Plant breeding and genetic engineering: A perspective", pages 17-21**

**RADIATION BOTANY, vol. 15, 1975, Pergamon Press (GB), P.J. BOTTINO: "The potential of genetic manipulation in plant cell cultures for plant breeding", pp. 1-16**

(73) Titulaire: **LABORATOIRE D'AMELIORATION DES PLANTES DE L'UNIVERSITE PARIS XI (ORSAY)**
**F-91405 Orsay (FR)**
(73) Titulaire: **ASSOCIATION DES CREATEURS DE VARIETES FOURRAGERES (ACVF)**
**7, rue Coq-Héron**
**F-75001 Paris (FR)**

(72) Inventeur: **Teoule, Evelyne**
**1 Square D. Papin Parc Montaigne**
**F-78330 Fontenay-le-Fleury (FR)**
Inventeur: **Dattee, Yvette**
**5 Bld du Montparnasse**
**F-75006 Paris (FR)**

(74) Mandataire: **Martin, Jean-Jacques et al**
**Cabinet REGIMBEAU 26, Avenue Kléber**
**F-75116 Paris (FR)**

Courier Press, Leamington Spa, England.

(56) Documents cités:

**CHEMICAL ABSTRACTS, vol. 95, no. 5, 3 aou7t 1981, page 420, résumé no. 39193b, COLUMBUS, OHIO (US) A.V. MEZENTSEV:"Mass plant regeneration from alfalfa cells and protoplasts."**

**CHEMICAL ABSTRACTS, vol. 96, no. 19, 10 mai 1982, page 262, résumé no. 157258v, COLUMBUS, OHIO (US) A.V. MEZENTSEV:"Experimental morphogenesis and regeneration of Medicago sp. from somatic cells."**

**Zeitschrift für Naturforschung, Band 28c, Heft 11/12, Nov./Déc. 1973, pages 737-741**

**Zeitung für Pflanzenphysiologie, 1982 (197), 59-63**

**Zeitung für Pflanzenphysiologie, 1980, (99), 261-270**

**Zeitung für Pflanzenphysiologie, 1982, (106), 105-110**

**Zeitung für Pflanzenphysiologie, 1982, (107), 231-235**

**Zeitung für Pflanzenphysiologie, 1980, (96), 135-141**

**Plant Science Letters, 1981, (20), 297-304**

## Description

La présente invention concerne la préparation d'hybrides somatiques de luzernes par fusion de protoplastes de deux espèces de luzernes, notamment de Medicago falcata avec Medicago sativa, la luzerne cultivée.

Les produits de fusion de ces deux espèces permettent d'introduire une variabilité génétique chez la luzerne conduisant à la sélection de nouvelles variétés industriellement intéressantes.

Ceci est particulièrement important car, à la différence du maïs par exemple, les hybrides de luzernes commercialement utilisables sont difficiles à obtenir. Les méthodes de sélection de la luzerne sont compliquées du fait des effets de consanguinité, de l'allogamie et de la tétraploïdie. Cette difficulté de la sélection explique en partie pourquoi les rendements en maïs ont augmenté de 320% en 45 ans alors que les rendements en luzerne n'augmentaient dans le même temps que de 40% comme cela est rappelé dans Bioscience, vol. 30, No. 1, 1980, 17—21.

Par ailleurs, les schémas de sélection classiques utilisent toujours du matériel issu des mêmes populations et il peut sembler utile d'introduire des géniteurs possédant d'autres caractéristiques et qui augmenteraient la variabilité génétique.

En effet, la luzerne est une plante relativement sensible à certaines conditions de milieu à des maladies et à des insectes; l'obtention d'hybrides somatiques peut permettre d'obtenir des luzernes plus résistantes et d'augmenter les rendements.

Bien que l'on sache régénérer des plantes à partir de protoplastes de M. sativa, comme cela est décrit par exemple par A. V. Mezentsev, Chem. Abs. vol. 95, 1981, 420, 39193b et vol. 96, 1982, 262, 157258v, et que l'on sache effectuer des hybridations somatiques avec régénération sur certaines plantes telles que le tabac, comme cela est décrit dans le brevet des Etats-Unis d'Amérique No. 4 300 310, il n'existe aucun exemple de régénération après le traitement de fusion sur M. sativa et aucun exemple connu de fusion suivie de régénération chez les légumineuses.

La présente invention propose un procédé d'obtention d'hybrides somatiques de luzernes, caractérisé en ce que:

a) on isole les protoplastes de deux génotypes ou de deux espèces de luzernes dans un milieu de culture,

b) les protoplastes obtenus sont fusionnés dans un milieu de culture contenant un sel de calcium comme agent fusionnant,

c) après l'opération de fusion, on effectue la régénération des plantes.

Ce procédé peut également s'appliquer à la fusion de protoplastes de deux génotypes de la même espèce.

Les protoplastes de chaque génotype ou espèce sont préparés séparément par digestion enzymatique des tissus végétaux dans un milieu de culture dilué par exemple à l'aide de cellulase, hémicellulase et pectinase dans un milieu de culture dérivé de celui de Kao.

Un élément important de ce procédé est l'utilisation d'un sel de calcium, en particulier le chlorure de calcium comme agent fusionnant. De préférence la solution de fusion est exempte de polyéthylène glycol mais peut contenir par exemple du mannitol dans un tampon tel qu'un tampon glycine. En effet, il semble que les protoplastes de Medicago supportent mal les traitements de fusion mettant en oeuvre le PEG. Les protoplastes isolés sont mis en suspension dans la solution de fusion puis la suspension est placée à 32°C pendant environ 15 minutes.

Après centrifugation et lavage, les protoplastes issus de l'opération de fusion sont incubés à l'obscurité pendant une dizaine de jours.

Les protoplastes sont ensuite cultivés en lumière diffuse sur un milieu de culture dérivé de celui de Kao par apport régulier de milieu frais.

Les embryoïdes obtenus sont alors cultivés sur papier filtre puis repiqués sur milieu solide.

On obtient ainsi, après un délai d'environ 6 mois, des plantules qui sont soumises à a l'étude pour rechercher les caractéristiques les différenciant des parents.

Exemple 1

On utilise comme parents deux espèces de luzerne:

— Medicago sativa, et
— Medicago falcata.

Les protoplastes sont obtenus par une incubation lente de 15 à 16 heures à l'obscurité à 26°C, dans une solution enzymatique contenant: cellulase, hémi-cellulase et pectinase, diluée avec du milieu de culture, dérivé de celui de Kao (Molec. Gen. Genet. 150, 225—230, 1977).

| | |
|---|---|
| $NH_4NO_3$ | 600,00 mg/l |
| $KNO_3$ | 1900,00 |
| $CaCl_2 . 2H_2O$ | 600,00 |
| $MgSO_4 . 7H_2O$ | 300,00 |
| $KH_2PO_4$ | 170,00 |
| KCl | 300,00 |
| | |
| KI | 0,75 mg/l |
| $H_3BO_3$ | 3,00 |
| $MnSO_4 . H_2O$ | 10,00 |
| $ZnSO_4 . 7H_2O$ | 2,00 |
| $Na_2MoO_4 . 2H_2O$ | 0,25 |
| $CuSO_4 . 5H_2O$ | 0,025 |
| $CoCl_2 . 6H_2O$ | 0,025 |
| Pour EDTA: 8 ml/l. | |
| | |
| Inositol | 100,00 mg/l |
| Nicotinamide | 1,00 |
| Pyridoxine HCl | 1,00 |
| Thiamine HCl | 10,00 |
| D-Ca pantothénate | 0,50 |
| Acide folique | 0,20 |
| Acide p-aminobenzoïque | 0,01 |
| | |
| Biotine | 0,005 mg/l |
| Chlorure de choline | 0,50 |
| Riboflavine | 0,10 |
| Acide ascorbique | 1,00 |

| | |
|---|---|
| Vitamine A | 0,005 |
| Vitamine D$_3$ | 0,005 |
| Vitamine B$_{12}$ | 0,01 |
| | |
| Glucose | 110400,00 mg/l |
| Saccharose | 125,00 |
| Fructose | 125,00 |
| Ribose | 125,00 |
| Xylose | 125,00 |
| Mannose | 125,00 |
| Rhamnose | 125,00 |
| Cellobiose | 125,00 |
| Sorbitol | 125,00 |
| Mannitol | 125,00 |

Acides organiques (pH=5,5)

| | |
|---|---|
| Pyruvate de sodium | 5 mg/l |
| Acide citrique | 10 |
| Acide malique | 10 |
| Acide fumarique | 10 |
| | |
| 2,4 D | 0,2 mg/l |
| Zéatine | 0,5 |
| A.N.A. | 1,0 |
| | |
| N.Z. amine | 125 mg/l |
| | |
| Eau distillée | 1000 ml |
| | (pH=5,7/ |
| | NaOH filtré). |

La solution enzymatique est ôtée puis les protoplastes sont lavés une fois avec du milieu de culture et mis en suspension à 250 000 protoplastes/ml.

Les suspensions sont alors réparties comme suit:

1 tube avec 2 ml de protoplastes de M. sativa

1 tube avec 2 ml de protoplastes de M. falcata (témoins de viabilité des protoplastes)

1 tube avec 4 ml de protoplastes de M. sativa

1 tube avec 4 ml de protoplastes de M. falcata (témoins de viabilité après l'opération de fusion)

4 tubes avec 2 ml de protoplastes de M. sativa + 2 ml de protoplastes de M. falcata (destinés à l'opération d'allofusion).

Tous les tubes, sauf les témoins de viabilité des protoplastes, sont centrifugés à 100 g pendant 4 minutes; le surnageant est ôté. Puis, on ajoute dans chaque tube 2 ml de solution de fusion:

| | |
|---|---|
| CaCl$_2$ 2H$_2$O | 0,74 g |
| Mannitol | 9,5 g |

dans 100 ml de tampon glycine à 0,375 g de glycine pour 100 ml.

Le pH est ajusté à 10,4 et la solution est stérilisée par filtration.

Les protoplastes sont remis en suspension dans cette solution à la pipette Pasteur; puis les tubes sont mis dans un bain-marie à 32°C pendant 15 minutes.

Les tubes sont ensuite centrifugés pendant 5 minutes à 60 g. Les protoplastes sont alors lavés une fois avec une solution saline (sels de Frearson, Frearson et al., Dev. Biol. 33, 130—137 (1973):

| | |
|---|---|
| KH$_2$PO$_4$ | 27,2 mg/l |
| KNO$_3$ | 101,0 |
| CaCl$_2$ . 2H$_2$O | 1480,0 |
| MgSO$_4$ . 7H$_2$O | 246,0 |
| KI | 0,16 |
| CuSO$_4$ . 5H$_2$O | 0,025 |

et une fois avec du milieu de culture puis mis en suspension à 50 000 protoplastes/ml à raison de 3,5 ml par boîte, dans des boîtes de Pétri prétraitées (de 5 cm de diamètre) scellées au parafilm.

Les protoplastes sont cultivés à 26°C, à l'obscurité pendant 10 jours, puis en lumière diffuse.

A 7 jours de culture, on observe des divisions dans toutes les boîtes, on dilue alors de la façon suivante:

Les 4/5 du contenu de chaque boîte sont transférés dans une boîte neuve, on réajuste alors la quantité de liquide dans les boîtes par adjonction de milieu frais; (ce procédé limite les pertes dues à la production de phénols en cours de culture).

Les cultures sont ensuite mises en lumière diffuse et entretenues en ajoutant du milieu frais tous les 7 jours jusqu'à l'obtention de petits embyroïdes de 1 mm qui sont alors cultivés sur papier filtre jusqu'au repiquage en milieu solide.

Le pourcentage de fusion, délicat à observer du fait que la manipulation s'effectue en tube, peut être estimé entre 5 et 10%.

Les plantes régénérées sont étudiées pour un certain nombre de marqueurs: cytoplasmiques (la stérilité mâle), nucléaires (marqueurs enzymatiques, biométrie de la plante, coloration des fleurs). Il est ainsi possible de détecter les produits d'allofusion. De cette façon on a pu mettre en évidence différents hybrides.

Plusieurs hybrides somatiques suffisamment développés ont pu être identifiés comme étant des produits de fusion par le zymogramme des peroxydases et des B amylases qui cumulent les bandes des deux génotypes parentaux et par la bigarrure des fleurs qui caractérisé les hybridations entre M. sativa et M. falcata. Pour d'autres plantes, on a pu montrer par analyse enzymatique qu'il s'agissait d'hybrides somatiques partiels.

**Revendications**

1. Procédé d'obtention d'hybrides somatiques de luzernes, caractérisé en ce que:

a) on isole les protoplastes de deux génotypes ou de deux espèces de luzernes dans un milieu de culture,

b) les protoplastes obtenus sont fusionnés dans un milieu de culture contenant un sel de calcium comme agent fusionnant,

c) après l'opération de fusion, on effectue la régénération des plantes.

2. Procédé selon la revendication 1, caractérisé en ce que la solution de fusion contient du chlorure de calcium mais est exempte de polyéthylène glycol.

3. Procédé selon l'une des revendications 1 ou 2, caractérisé en ce que les protoplastes sont préparés par digestion enzymatique de fragments de tissus végétaux dans un milieu de culture dilué.

4. Procédé selon l'une des revendications 1 à 3, caractérisé en ce que la fusion est effectuée par mise en suspension des protoplastes issus de l'étape a) dans une solution de fusion contenant du chlorure de calcium et du mannitol.

5. Procédé selon l'une des revendications 1 à 4, caractérisé en ce que la régénération est effectuée par mise en suspension des protoplastes fusionnés de l'étape b) dans un milieu de culture.

6. Procédé selon l'une des revendications 1 à 5, caractérisé en ce que le milieu de culture est le milieu de Kao ou un milieu qui en dérive.

7. Procédé selon l'une des revendications 1 à 6, caractérisé en ce que les deux espèces de luzernes parentes sont Medicago sativa et Medicago falcata.

**Patentansprüche**

1. Verfahren zur Gewinnung von somatischen Luzerne-Hybriden, dadurch gekennzeichnet, daß:

a) man die Protoplasten von zwei Luzerne-Genotypen oder -Sorten in einem Nährboden isoliert,

b) die erhaltenen Protoplasten in einem Nährboden verschmolzen werden, der als Verschmelzungsagens ein Calciumsalz enthält,

c) man nach dem Verschmelzungsschritt die Regeneration der Pflanzen bewirkt.

2. Verfahren nach Anspruch 1, dadurch gekennzeichnet, daß die Verschmelzungslösung Calciumchlorid enthält, aber frei ist von Polyethylenglykol.

3. Verfahren nach einem der Ansprüche 1 oder 2, dadurch gekennzeichnet, daß die Protoplasten durch enzymatische Verdauung von Pflanzengewebefragmenten in einem verdünnten Nährboden hergestellt werden.

4. Verfahren nach einem der Ansprüche 1 bis 3, dadurch gekennzeichnet, daß die Verschmelzung dadurch bewirkt wird, daß die im Schritt a) gewonnenen Protoplasten in einer Verschmelzungslösung suspendiert werden, die Calciumchlorid und Mannit enthält.

5. Verfahren nach einem der Ansprüche 1 bis 4, dadurch gekennzeichnet, daß die Regeneration dadurch bewirkt wird, daß die verschmolzenen Protoplasten aus dem Schritt b) in einem Nährboden suspendiert werden.

6. Verfahren nach einem der Ansprüche 1 bis 5, dadurch gekennzeichnet, daß der Nährboden der Kao-Nährboden oder ein Nährboden, der sich davon ableitet, ist.

7. Verfahren nach einem der Ansprüche 1 bis 6, dadurch gekennzeichnet, daß die zwei Sorten von Stamm-Luzernen Medicago sativa und Medicago falcata sind.

**Claims**

1. A process for the production of somatic hybrids of lucernes, characterised in that:

(a) the protoplasts of two genotypes or of two species of lucerne are isolated in a culture medium,

(b) the resulting protoplasts are fused in a culture medium containing a calcium salt as fusion agent,

(c) after the fusion operation regeneration of the plants is effected.

2. A process according to claim 1, characterised in that the fusion solution contains calcium chloride but contains no polyethylene glycol.

3. A process according to claim 1 or 2, characterised in that the protoplasts are prepared by enzymatic digestion of fragments of vegetable tissue in a dilute culture medium.

4. A process according to any one of claims 1 to 3, characterised in that the fusion is effected by suspending the protoplasts resulting from step (a) in a fusion solution containing calcium chloride and mannitol.

5. A process according to any one of claims 1 to 4, characterised in that the regeneration is effected by suspending the fused protoplasts of step (b) in a culture medium.

6. A process according to any one of claims 1 to 5, characterised in that the culture medium is Kao medium or a medium derived therefrom.

7. A process according to any one of claims 1 to 6, characterised in that the two parent lucerne species are Medicago sativa and Medicago falcata.